# EUROPEAN PATENT APPLICATION

(11) **EP 3 919 090 A1**
(43) Date of publication of application: **08.12.2021**
(21) Application number: 21177727.1
(22) Date of filing: 04.06.2021
(51) Int. Cl.: A61L 9/20, A41D 13/11, A42B 3/28, A42B 3/22, A62B 18/00, A62B 18/02

(54) **AIR STERILIZATION MASK**

(30) Priority: 05.06.2020 TW 109207102 U
(71) Applicant: Lin, Che-hung, Taoyuan City 334026 (TW)
(72) Inventor: Lin, Che-hung, Taoyuan City 334026 (TW)
(74) Representative: dompatent von Kreisler Selting Werner - Partnerschaft von Patent- und Rechtsanwälten mbB

(57) **Abstract**

An air sterilization mask (10, 20, 30, 40) including main body (100) and air sterilization component (200). Main body (100) has accommodation space (110). Air sterilization component (200) includes casing (210), airflow generating unit (220) and light sterilizer (230). Casing (210) has inlet end (211), outlet end (212), top opening (2141), bottom opening (2171) and channel (213). Main body (100) is selectively connected to one of inlet and outlet ends (211, 212). Top opening (2141) is located on inlet end (211). Bottom opening (2171) is located on outlet end (212). Two opposite sides of channel (213) are respectively in fluid communication with top opening (2141) and bottom opening (2171). Accommodation space (110) and outside environment are in fluid communication with each other via channel (213). Airflow generating unit (220) is selectively disposed in one of inlet end (211), outlet end (212), and channel (213). Light sterilizer (230) is disposed in casing (210) and light emitted from light sterilizer (230) is irradiated toward at least a part of channel (213).

## Description

### CROSS-REFERENCE TO RELATED APPLICATIONS

### Technical Field

The disclosure relates to a mask, more particularly to an air sterilization mask including an airflow generating unit and a light sterilizer.

### Background

Due to the population growth, infectious disease (e.g., flu and Tuberculosis) can be spread rapidly in areas having high population density, such as urban area. In order to reduce the risk of the infection of such infectious disease, personal protective equipment (PPE) (e.g., goggles and surgical masks) are developed and widely used in hospital, quarantine station or even in daily life.

However, some types of PPE are unable to effectively block such infectious disease away from the wearer. Although some other types of PPE can effectively block such infectious disease in the air from the wear, the such PPE is not quite breathable, making the wearer unable to breath comfortably. In addition, most of the PPE are disposable and thus generate waste of resources. Thus, it is desired to develop a PPE that can effectively block the infectious disease from the wearer, allow the wearer to breath comfortably and reduce the disposable waste.

### SUMMARY

The disclosure provides an air sterilization mask that can effectively block the infectious disease away from the wearer, allow the wearer to breath comfortably, and reduce the disposable waste.

One embodiment of this disclosure provides an air sterilization mask including a main body and at least one air sterilization component. The main body has an accommodation space. The at least one air sterilization component includes a casing, an airflow generating unit and a light sterilizer. The casing has an inlet end, an outlet end, a top opening, a bottom opening and a channel. The main body is selectively connected to one of the inlet end and the outlet end. The top opening is located on the inlet end. The bottom opening is located on the outlet end. Two opposite sides of the channel are respectively in fluid communication with the top opening and the bottom opening. The accommodation space and an outside environment are in fluid communication with each other via the channel. The airflow generating unit is selectively disposed in one of the inlet end, the outlet end, and the channel. The light sterilizer is disposed in the casing and a light emitted from the light sterilizer is irradiated toward at least a part of the channel.

According to the air sterilization mask disclosed by the above embodiments, since the air sterilization mask includes the light sterilizer and the airflow generating unit, the air inhaled into or exhausted from the accommodation space of the main body is sterilized by the ultraviolet light emitted from the light sterilizer. Also, with the airflow that is generated by the airflow generating unit and circulates between the accommodation and the outside environment, the air in the accommodation space is prevented from being sultry. Therefore, the air sterilization mask still can effectively sterilize the air and allow the wearer to breath comfortably. In addition, the air sterilization mask can be reused so as to achieve the reduction of the disposable waste.

### BRIEF DESCRIPTION OF THE DRAWINGS

The present disclosure will become better understood from the detailed description given herein below and the accompanying drawings which are given by way of illustration only and thus are not intending to limit the present disclosure and wherein:
FIG. 1 is a perspective view of an air sterilization mask according to a first embodiment of the disclosure;
FIG. 2 is an exploded perspective view of an air sterilization component of the air sterilization mask in FIG. 1;
FIG. 3A is a cross-sectional view of the air sterilization component of the air sterilization mask in FIG. 1;
FIG. 3B is a cross-sectional view of an air sterilization component according to another embodiment of the disclosure;
FIG. 4 shows the operation of the air sterilization component of the air sterilization mask in FIG. 1;
FIG. 5 shows another operation of the air sterilization component of the air sterilization mask in FIG. 1;
FIG. 6 is a perspective view of an air sterilization mask according to a second embodiment of the disclosure;
FIG. 7A is a front view of an air sterilization mask according to a third embodiment of the disclosure;
FIG. 7B is a partially enlarged cross-sectional view of the air sterilization mask in FIG. 7A;
FIG. 7C is another partially enlarged cross-sectional view of the air sterilization mask in FIG. 7A; and
FIG. 8 is a front view of an air sterilization mask according to a fourth embodiment of the disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

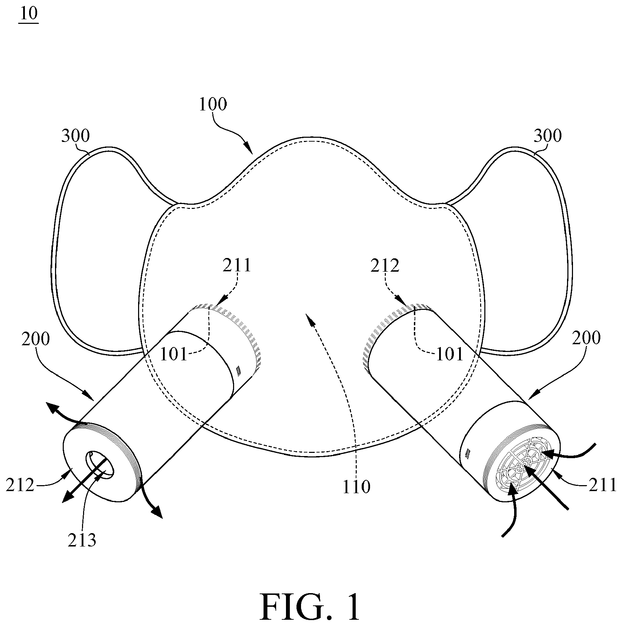

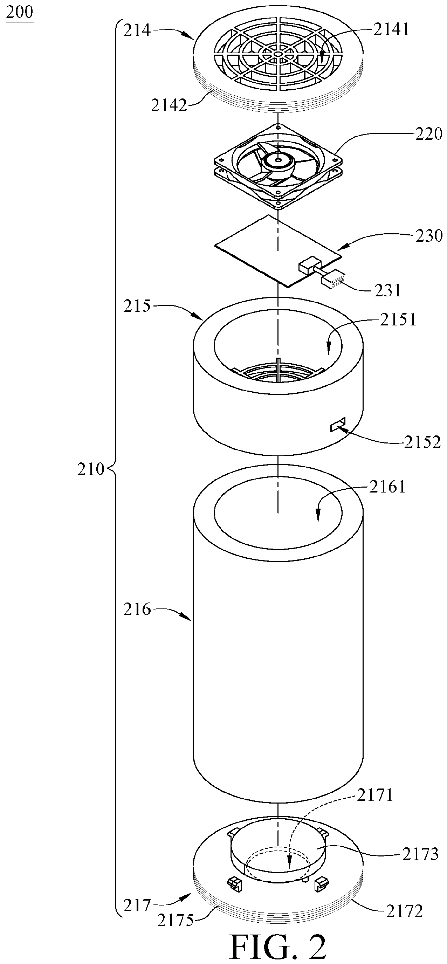

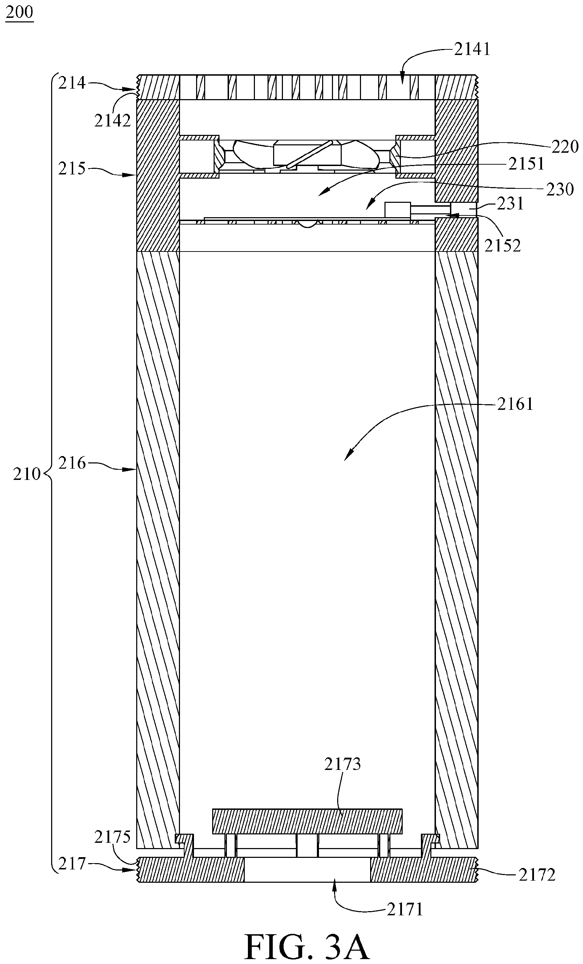

Please refer to FIGS. 1, 2 and 3A, where FIG. 1 is a perspective view of an air sterilization mask 10 according to a first embodiment of the disclosure, FIG. 2 is an exploded perspective view of an air sterilization component 200 of the air sterilization mask 10 in FIG. 1, and FIG. 3A is a cross-sectional view of the air sterilization component 200 of the air sterilization mask 10 in FIG. 1.

In this embodiment, the air sterilization mask 10 includes a main body 100, two air sterilization components 200 and two fasteners 300. In this embodiment, the main body 100 is configured to cover a part of a face of a wearer. Specifically, in this embodiment, the main body 100 is configured to cover a mouth and a nose of the wearer, but the disclosure is not limited thereto. In other embodiments, the main body may cover the whole face or a head of the wearer. The main body 100 has an accommodation space 110 that is configured to accommodate the mouth and the nose of the wearer. In this embodiment, the main body 100 is in tight contact with a skin of the wearer. In this embodiment, the accommodation space 110 is in fluid communication with the outside environment via the two air sterilization components 200. The detail structure and the operation of the air sterilization mask 10 will be described hereinafter.

The main body 100 may be made of rigid materials (e.g., glass and metal) or elastic materials (e.g., elastic resins). The main body 100 may be entirely or partially made from a light-permeable material. In such a case, the facial appearance, the facial expression, the facial makeup, and the lip language of the wearer wearing the air sterilization mask 10 can still be recognized, which allow the air sterilization mask 10 to be used in countries enacting the anti-mask law. In this embodiment, the main body 100 has two engagement structures 101. The engagement structures 101 are, for example, screw holes and in fluid communication with the accommodation space 110.

As shown in FIG. 2, the air sterilization component 200 includes a casing 210, an airflow generating unit 220 and a light sterilizer 230. The casing 210 has an inlet end 211, an outlet end 212, a top opening 2141, a bottom opening 2171 and a channel 213. Two opposite sides of the channel 213 are respectively connected to the inlet end 211 and the outlet end 212. The casing 210 includes a top cover 214, a top body 215, a bottom body 216 and a bottom cover 217. As shown in FIG. 3A, the top cover 214, the top body 215, the bottom body 216 and the bottom cover 217 are sequentially connected to one another. In addition, the top body 215 is removably disposed on the bottom body 216. The top opening 2141 is located on the inlet end 211, and the inlet end 211 is located on the top cover 214. The top body 215 has a top space 2151 and an insertion hole 2152 that are connected to each other. The bottom body 216 has a bottom space 2161. The bottom opening 2171 is located on the outlet end 212 and the outlet end 212 is located on the bottom cover 217. The top opening 2141, the top space 2151, the bottom space 2161 and the bottom opening 2171 are sequentially connected to one another so as to form the channel 213.

The main body 100 is selectively connected to the inlet end 211 and the outlet end 212 of the casing 210. Specifically, both of the bottom cover 217 and the top cover are able to be removably disposed on the main body 100. In detail, the top cover 214 has a top engagement structure 2142, and the bottom cover 217 has a bottom engagement structure 2175. The top engagement structure 2142 and the bottom engagement structure 2175 are, for example, screw threads, and are able to be screwed into the engagement structure 101 of the main body 100, thereby allowing the casing 210 of the air sterilization component 200 to be installed on the main body 100.

In this embodiment, the air sterilization mask 10 is in an exhaustion-inhalation mode. In detail, please refer to FIGS. 2 and 3A and further refer to the left part of FIG. 1, with respect to the air sterilization component 200 shown in the left part of FIG. 1, the top engagement structure 2142 is screwed into the engagement structure 101 of the main body 100 to directly connect the inlet end 211 of the casing 210 with the main body 100, such that the accommodation space 110 of the main body 100 is in fluid communication with the outside environment via the channel 213 and outlet end 212 of the casing 210.

Please refer to FIGS. 2 and 3A and further refer to the right part of FIG. 1, with respect to the air sterilization component 200 shown in the right part of FIG. 1, the bottom engagement structure 2175 is screwed into the engagement structure 101 of the main body 100, to directly connect the outlet end 212 with the main body 100, such that the accommodation space 110 of the main body 100 is in fluid communication with the outside environment via the channel 213 and the inlet end 211 of the casing 210.

Note that in other embodiments the top cover and the bottom cover may be removably disposed on the main body via press-fit, snap-fit, clips and the like.

Please refer to FIGS. 2 and 3A, the airflow generating unit 220 and the light sterilizer 230 are located in the top space 2151, and the airflow generating unit 220 is located closer to the top cover 214 than the light sterilizer 230. The light sterilizer 230 is, for example, an ultraviolet emitting device. There is no component or structure disposed between the light sterilizer 230 and the bottom space 2161, such that the ultraviolet light emitted from the light sterilizer 230 can be irradiated into the bottom space 2161 without being blocked, thereby allowing the ultraviolet light emitted from the light sterilizer 230 to effectively sterilize the air in the bottom space 2161. Note that in other embodiments, there may be one or more components or structures that are light-permeable disposed between the light sterilizer and the bottom space.

Please refer to FIGS. 2 and 3A, the bottom cover 217 further includes a cover body 2172 and a blocking sheet 2173. The bottom opening 2171 is formed on the cover body 2172. The blocking sheet 2173 is disposed on a part of the cover body 2172 and spaced apart from another part of the cover body 2172. Also, the blocking sheet 2173 covers the bottom opening 2171 to avoid ultraviolet light from leaking from the bottom opening 2171.

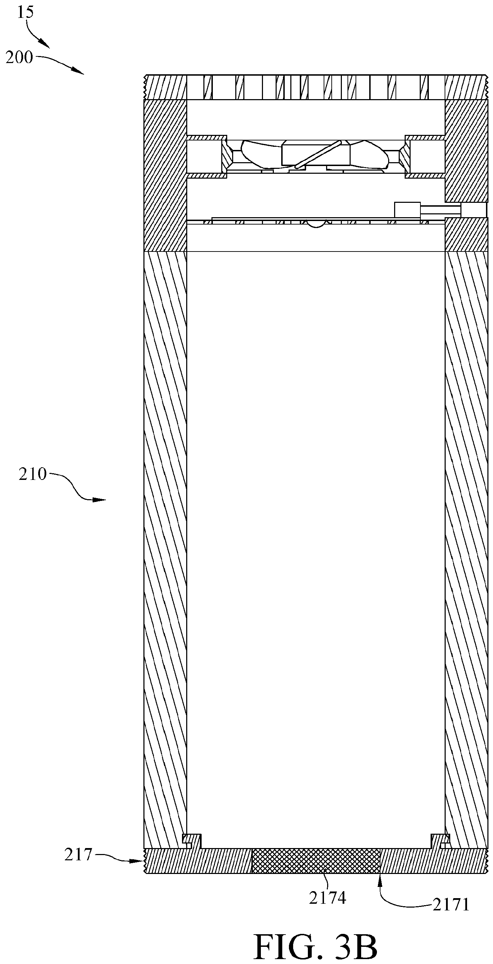

Please refer to FIG. 3B. FIG. 3B is a cross-sectional view of an air sterilization component according to another embodiment of the disclosure. The embodiment shown in FIG. 3B provides an air sterilization mask 15. Note that only a part of the air sterilization mask 15 is shown in FIG. 3B for brevity. In the air sterilization mask 15, a bottom cover 217 includes a cover body 2172 and a filter 2174. A bottom opening 2171 is located on the cover body 2172. The filter 2174 is disposed on the cover body 2172 and fully occupies the entire bottom opening 2171, thereby ensuring the air to pass through the filter 2174 before flowing out of the air sterilization component 200. The filter 2174 may be mesh filter, filtering sponge and the like. The filter 2174 can retain the infectious agents that are not killed by ultraviolet light on itself, and then the ultraviolet light can be irradiated on the filter 2174 to kill the infectious agents.

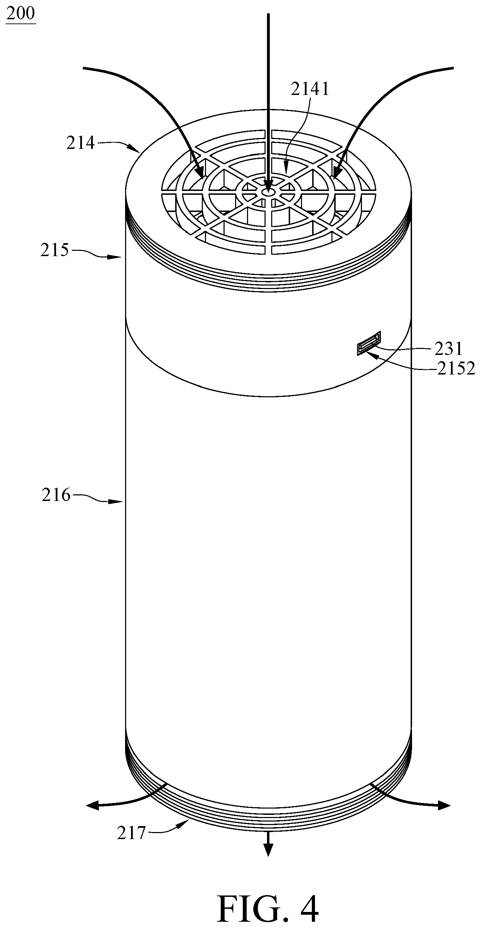

Please refer to FIGS. 2, 3A and 4, where FIG. 4 shows the operation of the air sterilization component of the air sterilization mask in FIG. 1. In this embodiment, as shown in FIGS. 3A and 4, the airflow generating unit 220 is disposed in the top body 215 and is located in the top space 2151. Note that in other embodiments, the airflow generating unit may be disposed on the inlet end, the outlet end or in the channel. The airflow generating unit 220 sucks air into the channel 213 of the casing 210 through the inlet end 211 of the air sterilization component 200, and blows it out of the channel 213 of the casing 210 through the outlet end 212. In this embodiment, the airflow generating unit 220 is, for example, a fan, but the disclosure is not limited thereto. In other embodiments, the airflow generating unit 220 may be an ion wind generator or the like. In this embodiment, the airflow generating unit 220 is located in the top space 2151, but the disclosure is not limited thereto. In other embodiments, the airflow generating unit may be located in the bottom space, the top cover or the bottom cover.

Please refer to FIGS. 2 and 3A, the light sterilizer 230 is, for example, a printed circuit board assembly including at least one power supply, circuit board and light emitting component. In this embodiment, the light sterilizer 230 is, for example, an ultraviolet emitting device including at least one battery, circuit board having driving circuit, ultraviolet emitter and charging connector. The ultraviolet light emitted from the light sterilizer 230 is irradiated toward at least a part of the channel 213. That is, the ultraviolet light emitted from the light sterilizer 230 passed to the at least a part of the channel 213 without being blocked. The ultraviolet light emitted from the light sterilizer 230 is directly irradiated to the bottom space 2161, and the light sterilizer 230 is electrically connected to the airflow generating unit 220. The light sterilizer 230 has a charging plug 231 disposed through the insertion hole 2152. The charging plug 231 may be direct current (DC) plug, universal serial bus (USB) plug, Micro USB plug and the like. With the charging plug 231, the air sterilization component 200 can be electrically connected to an external power supply (not shown) via the charging plug 231 disposed through the insertion hole 2152, and the power provided by the external power supply drives the light sterilizer 230 and the airflow generating unit 220 or charges the built-in battery of the light sterilizer 230.

In this embodiment, the light sterilizer 230 is disposed in the top space 2151, but the disclosure is not limited thereto. As long as the ultraviolet light emitted from the light sterilizer can be irradiated to at least a part of the channel, the light sterilizer may be disposed on the blocking sheet or any suitable portion of the bottom body. Additionally, in other embodiments, as long as the charging plug can be disposed through the insertion hole of the top body, the insertion hole may be located on the top cover, the bottom body or the bottom cover.

As shown in FIG. 1, the two fasteners 300 are respectively disposed on two opposite sides of the main body 100. In this embodiment, the two fasteners 300 are, for example, ropes, and are respectively configured to fixed the main body 100 to the ears of the wearer. Note that in other embodiments, the two fastener may be belts, hooks, velcro and the like, and may be configured to fix the main body to the head or other body parts of the wearer.

Hereinafter, the operation of the two air sterilization components 200 will be described. As shown in FIGS. 3A and 4, with respective to each air sterilization component 200, when the air sterilization component 200 is in operation, the airflow generating unit 220 sucks air into the channel 213 of the casing 210 through the inlet end 211 (top opening 2141) of the air sterilization component 200, and forces it to pass through the top space 2151 and the bottom space 2161, and then the air flows out of the channel 213 of the casing 210 through the outlet end 212 (bottom opening 2171) of the air sterilization component 200. It is noted that before the air flows out of the air sterilization component 200, the air is sterilized in the bottom space 2161 by the ultraviolet light emitted by the light sterilizer 230.

Please refer to FIGS. 2 and 3A and further refer to the left part of FIG. 1, with respect to the air sterilization component 200 that exhausts the air, the inlet end 211 of the casing 210 is directly connected to the main body 100, and the accommodation space 110 of the main body 100 is in fluid communication with the outside environment via the channel 213 and the outlet end 212 of the casing 210. The air exhausted by the wearer wearing the air sterilization mask 10 is sucked into the channel 213 of the casing 210 by the airflow generating unit 220 and then is sterilized by the ultraviolet light emitted from the light sterilizer 230. In this way, the air sterilization mask 10 can exhaust such sterilized air to the outside environment.

The exhaustion of the air sterilization component 200 is suitable for the infected wearer to prevent the infectious agents spread from the inflected wearer from being spread to the outside environment. By wearing the air sterilization mask 10 with the air sterilization component 200 exhausting the air, the air exhausted from the infected wearer can be sterilized before further exhausted to the outside environment. Further, the exhaustion of the air sterilization component 200 is particularly suitable for the infected wearer in a case that there is no available negative pressure room in the hospital.

Please refer to FIGS. 2 and 3A and further refer to the right part of FIG. 1, with respect to the air sterilization component 200 that inhales air, the outlet end 212 is directly connected to the main body 100, and the accommodation space 110 of the main body 100 is in fluid communication with the outside environment via the channel 213 and the inlet end 211 of the casing 210. The air inhaled from the outside environment into the air sterilization mask 10 is sterilized by the ultraviolet light emitted from the light sterilizer 230. In this way, the wearer of the air sterilization mask 10 can inhale the sterilized air.

The inhalation of the air sterilization component 200 is suitable for a healthy wearer to prevent himself/herself from being infected by infectious agents in the outside environment. For example, when the healthy wearer takes public transportation, talks to other people or goes to hospital and thus contacts lots of people, the air sterilization mask 10 that inhales the air can sterilize the air in the outside environment that may contain infectious agents, and the wearer can inhale the sterilized air.

Further, the air is circulated between the accommodation space 110 of the main body 100 and the outside environment via the air sterilization components 200. Thus, although the main body 100 is in tight contact with a skin of the wearer, the air in the accommodation space 110 of the main body 100 is prevented from being sultry, and the air sterilization mask 10 still can effectively sterilize the air and allow the wearer to breath in a smooth manner.

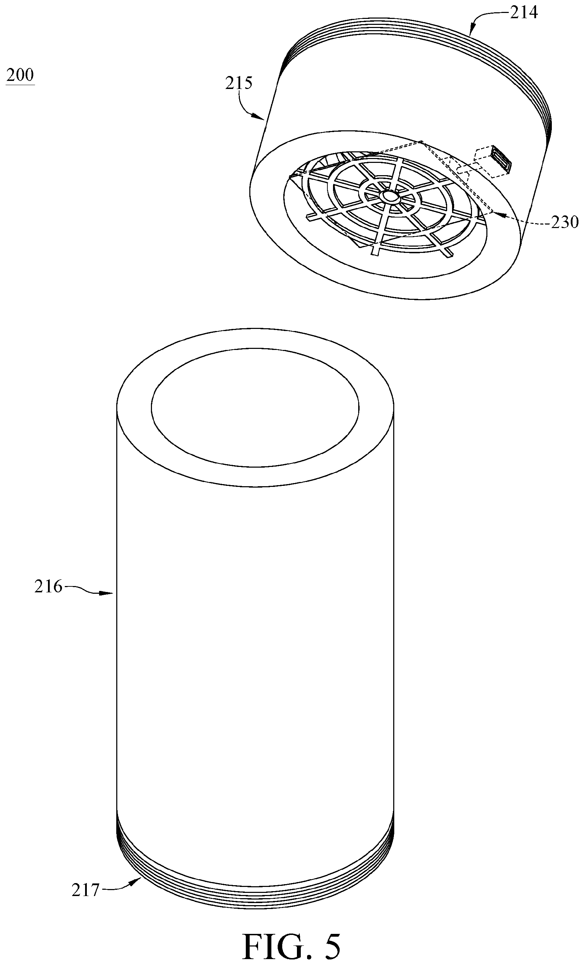

In addition, the air sterilization component 200 can operate in a manner independent from the main body 100. As shown in FIG. 4, the air sterilization component 200 is operated as an independent air sterilizer. In addition, please refer to FIG. 5, where FIG. 5 shows another operation of the air sterilization component of the air sterilization mask in FIG. 1. The top body 215 where the air sterilization component 200 is located can be separated from the bottom body 216, and can be served as an independent handheld ultraviolet emitting device.

Note that, in other embodiments, there may be a gap between the main body and the skin of the wearer, and the air may be circulated between the outside environment and the accommodation space of the main body via the gap between the main body and the skin of the wearer by the pressure difference produced by the airflow generating unit. Specifically, when the air sterilization component exhausts the air, the pressure of the accommodation space is smaller than the pressure of the outside environment, and thus the air is sucked into the accommodation space from the outside environment via the gap between the main body and the skin. When the air sterilization component inhales the air, the pressure of the accommodation space is greater than the pressure of the outside environment, and thus the air is exhausted into the outside environment from the accommodation space via the gap between the main body and the skin.

Also, the air sterilization mask 10 may be in an exhausting mode or an inhaling mode. That is, the inlet ends 211 or outlet ends 212 of the air sterilization components 200 may all be connected to the main body 100. In addition, in other embodiments, there may be only one air sterilization component that exhausts the air or inhales the air. Further, in other embodiments, there may be more than two air sterilization components, where a part of the air sterilization components may exhaust the air and the remaining part of the air sterilization components may inhale the air.

In this embodiment, the two air sterilization components 200 are respectively disposed on two opposite portions of the main body 100 that is located on the same side of the main body 100 and respectively covering the cheeks of the wearer, but the disclosure is not limited thereto. In other embodiments, the two air sterilization components may be disposed on the middle portion of the main body covering the mouth of the wearer, or may be disposed on the lower portion of the main body covering the chin of the wearer. Also, the two casing 210 of the two air sterilization components 200 are in a cylindrical shape, but the disclosure is not limited thereto. In other embodiments, the air sterilization components may be in a shape of square prism, rectangular prism or other types of prism.

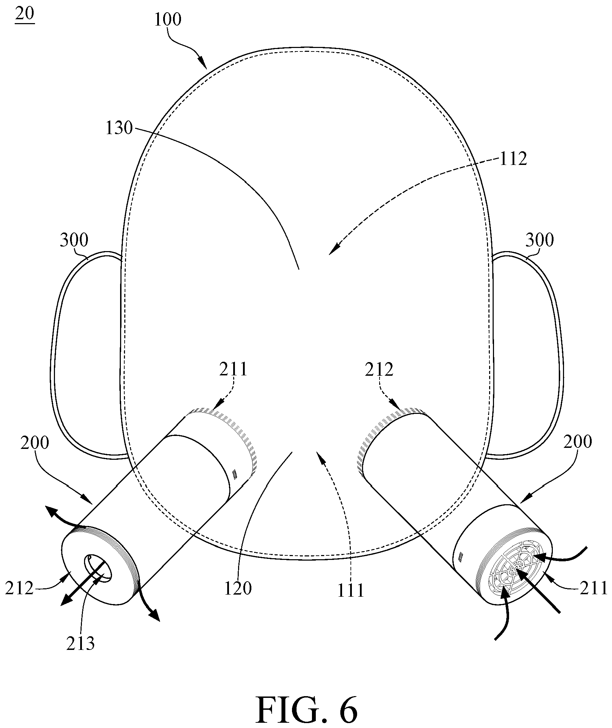

Please refer to FIG. 6. FIG. 6 is a perspective view of an air sterilization mask 20 according to a second embodiment of the disclosure. The main difference between the first embodiment and the second embodiment is the structure of the main body of the air sterilization mask, and thus only the main difference will be described hereinafter. The remaining part of the second embodiment can be understood through the descriptions made by referring to FIGS. 1, 2, 3A, 4 and 5. As shown in FIG. 6, in this embodiment, a main body 100 of the air sterilization mask 20 includes a mouth-nose covering part 120 and an eye-forehead covering part 130 that are connected to each other, where the mouth-nose covering part 120 is configured to cover the mouth and the nose of the wearer, and the eye-forehead covering part 130 is configured to cover the eyes and the forehead of the wearer. An accommodation space 110 of the main body 100 includes a mouth-nose accommodation space 111 and an eye-forehead accommodation space that are in fluid communication with each other, where the mouth-nose covering part 120 forms the mouth-nose accommodation space 111, and the eye-forehead covering part 130 forms the eye-forehead accommodation space. Two air sterilization components 200 are disposed on the mouth-nose covering part 120, and two channels 213 of the air sterilization components are in fluid communication with the mouth-nose accommodation space 111.

Besides the nose and the mouth of the wearer, the air sterilization mask 20 provided by the second embodiment covers the forehead and the eyes of the wearer, such that the infectious agents is further prevented from contacting the forehead and the eyes of the wearer. Thus, the air sterilization mask 20 can block the infectious agents away from the wearer in a more efficient manner.

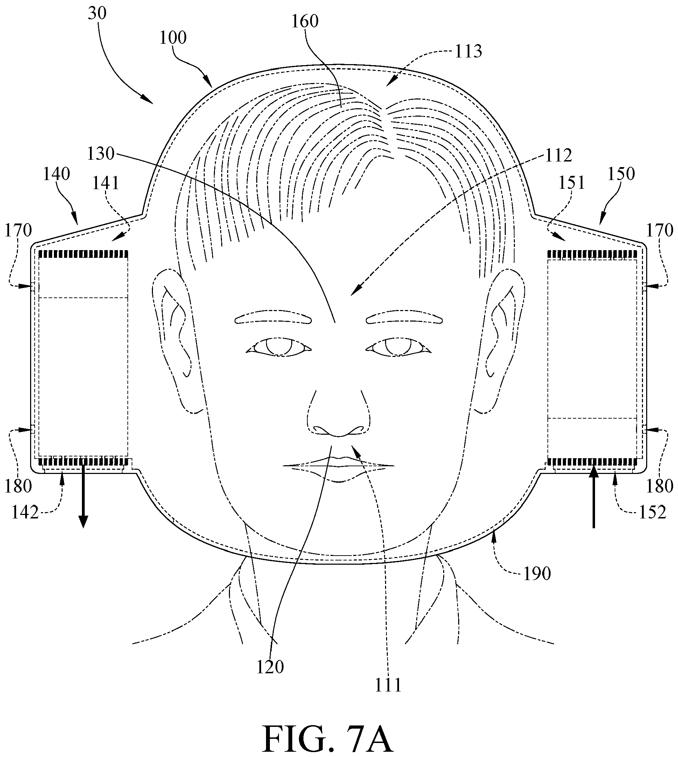

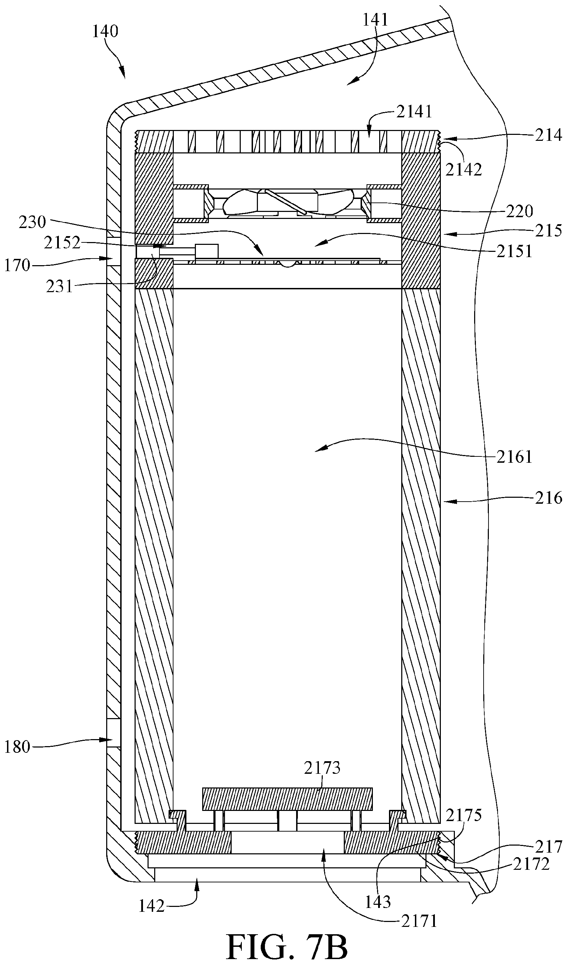

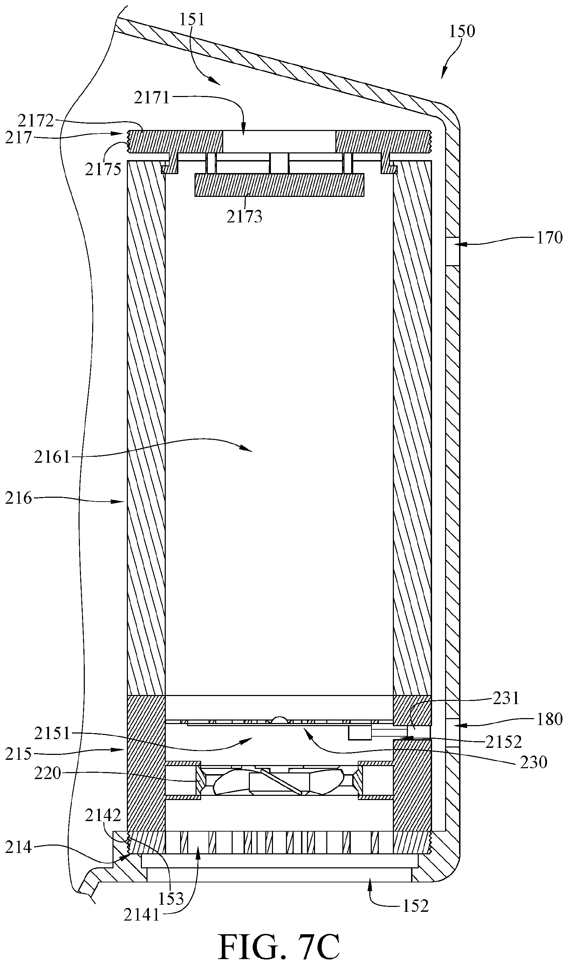

Please refer to FIGS. 7A, 7B and 7C. FIG. 7A is a front view of an air sterilization mask 30 according to a third embodiment of the disclosure. FIG. 7B is a partially enlarged cross-sectional view of the air sterilization mask 30 in FIG. 7A. FIG. 7C is another partially enlarged cross-sectional view of the air sterilization mask 30 in FIG. 7A. The main difference between the first embodiment and the third embodiment is the structure of the main body of the air sterilization mask and the position of the air sterilization components relative to the main body, and thus only the main difference will be described hereinafter. The remaining part of the third embodiment can be understood through the descriptions made by referring to FIGS. 1, 2, 3A, 4 and 5.

As shown in FIGS. 7A, 7B and 7C, in this embodiment, a main body 100 of the air sterilization mask 30 includes a mouth-nose covering part 120, an eye-forehead covering part 130, two side parts 140 and 150 and a top head-rear head covering part 160. The mouth-nose covering part 120 is configured to cover the mouth and the nose of the wearer. The eye-forehead covering part 130 is configured to cover the eyes and the forehead of the wearer. The top head-rear head covering part 160 is configured to cover the top and rear part of the head of the wearer. The two side parts 140 and 150 respectively cover the two opposite side parts of the face of the wearer, where two ears are respectively located on the two opposite side parts of the face. The two side parts 140 and 150 are respectively connected to two opposite sides of the mouth-nose covering part 120, two opposite sides of the eye-forehead covering part 130, and the two opposite sides of the top head-rear head covering part 160. The mouth-nose covering part 120, the eye-forehead covering part 130, the two side parts 140 and 150 and the top head-rear head covering part 160 together form an accommodation space 110. The accommodation space 110 includes a mouth-nose accommodation space 111, an eye-forehead accommodation space and a top head-rear head accommodation space 113 that are in fluid communication with one another. The mouth-nose covering part 120 forms the mouth-nose accommodation space 111. The eye-forehead covering part 130 forms the eye-forehead accommodation space 112. The two side parts 140 and 150 and the top head-rear head covering part 160 together form the top head-rear head accommodation space 113. The side part 140 has an installing recess 141 and a vent 142. The side parts 150 has an installing recess 151 and a vent 152. The installing recesses 141 and 151 are in fluid communication with the mouth-nose accommodation space 111, and the installing recess 141 is opposite to the installing recess 151.

Furthermore, the main body 100 further has a wearing opening 190 that is formed by the two side parts 140 and 150, the mouth-nose covering part 120 and the top head-rear head covering part 160 together. The wearing opening 190 is in fluid communication with the top head-rear head accommodation space 113.

In this embodiment, the air sterilization mask 30 includes two air sterilization components 200. The two air sterilization components 200 are respectively installed in the installing recesses 141 and 151. Two channels 213 of the two air sterilization component 200 are in fluid communication with the vents 142 and 152, respectively.

In this embodiment, the side part 140 has an engagement structure 143, and the side part 150 has an engagement structure 153. The engagement structures 143 and 153 are, for example, screw holes, and are respectively connected to the installing recesses 141 and 151. A top engagement structure 2142 and a bottom engagement structure 2175 of each air sterilization component 200 are, for example, screw threads. Both of the top engagement structure 2142 and the bottom engagement structure 2175 of the air sterilization component 200 that is installed in the installing recess 141 are able to be screwed into the engagement structures 143 of the side part 140. Both of the top engagement structure 2142 and the bottom engagement structure 2175 of the air sterilization component 200 that is installed in the installing recess 151 are able to be screwed into the engagement structures 153 of the side part 150.

In this embodiment, as shown in FIG. 7B, with respect to the air sterilization component 200 that is installed in the installing recess 141, the bottom engagement structure 2175 of the air sterilization component 200 is screwed into the engagement structures 143 of the side part 140, and a bottom cover 217 (outlet end) of the air sterilization component 200 is located closer to the vent 142 than a top cover 214 (inlet end) of the air sterilization component 200. Also, as shown in FIGS. 7A and 7B, with respect to the air sterilization component 200 that is installed in the installing recess 141, an airflow generating unit 220 of the air sterilization component 200 blows an air to the outside environment from the mouth-nose accommodation space 111, the eye-forehead accommodation space and the top head-rear head accommodation space 113 through the vent 142. That is, the air sterilization component 200 exhausts the air. Furthermore, during the exhaustion of the air sterilization component 200, the air can be inhaled from the outside environment into the accommodation space 100 via the wearing opening 190 due to the pressure difference therebetween.

Also, in this embodiment, as shown in FIG. 7C, with respect to the air sterilization component 200 that is installed in the installing recess 151, the top engagement structure 2142 of the air sterilization component 200 is screwed into the engagement structures 153 of the side part 150, and a top cover 214 (inlet end) of the air sterilization component 200 is located closer to the vent 152 than a bottom cover 217 (outlet end) of the air sterilization component 200. Also, as shown in FIGS. 7A and 7C, with respect to the air sterilization component 200 that is installed in the installing recess 151, an airflow generating unit 220 of the air sterilization component 200 sucks an air into the mouth-nose accommodation space 111, the eye-forehead accommodation space and the top head-rear head accommodation space 113 from the outside environment through the vent 152. That is, the air sterilization component 200 inhales the air. Furthermore, during the inhalation of the air sterilization component 200, the air can be exhausted to the outside environment from the accommodation space 100 via the wearing opening 190 due to the pressure difference therebetween.

Note that in other embodiments, the air sterilization components may be disposed on the top head-rear head covering part.

In this embodiment, each side part 140 and 150 of the air sterilization mask 30 further includes a first insertion hole 170 and a second insertion hole 180 that are configured for the penetration of a charging plug.

As shown in FIG. 7B, in the side part 140 where the air sterilization component 200 that exhausts the air is disposed, the first insertion hole 170 and the second insertion hole 180 are connected to the installing recess 141, the first insertion hole 170 is located on a side of the second insertion hole 180 that is located away from the vent 142, and the first insertion hole 170 is located closer to an insertion hole 2152 of the top body 215 than the second insertion hole 180. Thus, the first insertion hole 170 can be aligned with an insertion hole 2152 of the top body 215 and configured for the penetration of the charging plug.

As shown in FIG. 7C, in the side part 150 where the air sterilization component 200 that inhales the air is disposed, the first insertion hole 170 and the second insertion hole 180 are connected to the installing recess 151, the first insertion hole 170 is located on a side of the second insertion hole 180 that is located away from the vent 152, and the second insertion hole 180 is located closer to the insertion hole 2152 of the top body 215 than the first insertion hole 170. Thus, the second insertion hole 180 can be aligned with an insertion hole 2152 of the top body 215 and configured for the penetration of the charging plug.

In other embodiments, when the air sterilization mask is in the inhaling mode, the second insertion holes are respectively aligned with the two insertion holes of the two top bodies. In still other embodiments, when the air sterilization mask is in the exhausting mode, the first insertion holes are respectively aligned with the two insertion holes of the top bodies.

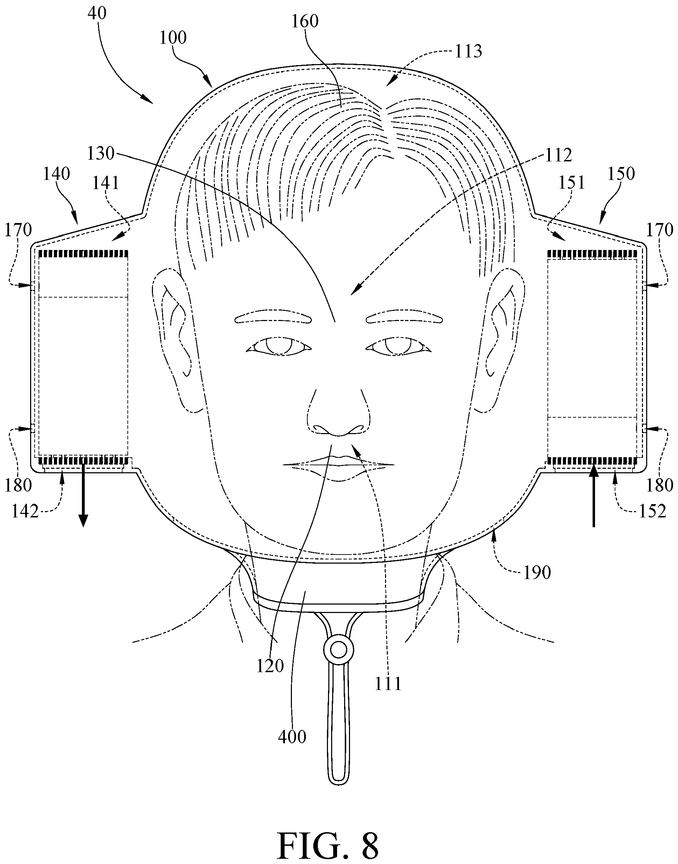

Please refer to FIG. 8, there is shown a front view of an air sterilization mask 40 according to a fourth embodiment of the disclosure. The main difference between the third embodiment and the fourth embodiment is that the air sterilization mask 40 of the fourth embodiment further includes a enclosing component 400, and thus only the main difference will be described hereinafter. The remaining part of the fourth embodiment can be understood through the descriptions made by referring to FIGS. 7A, 7B and 7C and further referring to FIGS. 1, 2, 3A, 4 and 5.

In this embodiment, the enclosing component 400 is disposed on the two edges of the main body 100 forming the wearing opening 190. In this embodiment, the enclosing component 400 includes a rope and a cord stopper slidably disposed on the rope, and the edges of the wearing opening 190 is enclosed by sliding the cord stopper. As the edges of the wearing opening 190 is bound, the outside environment is not in fluid communication with an accommodation space 110 of the main body. In other embodiments, the enclosing component may be a zipper, button or Velcro or any other fasteners that can allow the edges of the wearing opening to be detached from each other easily. In still other embodiments, the enclosing component may be adhesive or other fasteners that allow the edges of the wearing opening to be hardly detached from each other.

According to the air sterilization mask disclosed by the above embodiments, since the air sterilization mask includes the light sterilizer and the airflow generating unit, the air inhaled into or exhausted from the accommodation space of the main body is sterilized by the ultraviolet light emitted from the light sterilizer. Also, with the airflow that is generated by the airflow generating unit and circulates between the accommodation and the outside environment, the air in the accommodation space is prevented from being sultry. Therefore, the air sterilization mask still can effectively sterilize the air and allow the wearer to breath comfortably. In addition, the air sterilization mask can be reused so as to achieve the reduction of the disposable waste.

It will be apparent to those skilled in the art that various modifications and variations can be made to the present disclosure. It is intended that the specification and examples be considered as exemplary embodiments only, with a scope of the disclosure being indicated by the following claims and their equivalents.

## Claims

1. An air sterilization mask (10, 20, 30, 40), comprising:
a main body (100), having an accommodation space (110) (110); and
at least one air sterilization component (200), comprising:
a casing (210), wherein the casing (210) has an inlet end (211), an outlet end (212), a top opening (2141) (2141), a bottom opening (2171) (2171) and a channel (213), the main body (100) is selectively connected to one of the inlet end (211) and the outlet end (212), the top opening (2141) (2141) is located on the inlet end (211), the bottom opening (2171) (2171) is located on the outlet end (212), two opposite sides of the channel (213) are respectively in fluid communication with the top opening (2141) (2141) and the bottom opening (2171) (2171), and the accommodation space (110) (110) and an outside environment are in fluid communication with each other via the channel (213);
an airflow generating unit (220) (220), selectively disposed in one of the inlet end (211), the outlet end (212), and the channel (213); and
a light sterilizer (230), wherein the light sterilizer (230) is disposed in the casing (210) and a light emitted from the light sterilizer (230) is irradiated toward at least a part of the channel (213).

2. The air sterilization mask (10, 20, 30, 40) according to claim 1, wherein the air sterilization mask (10, 20, 30, 40) has an inhaling mode and an exhausting mode, when the air sterilization mask (10, 20, 30, 40) is in the inhaling mode, the outlet end (212) of the at least one air sterilization component (200) is connected to the main body (100), when the air sterilization mask (10, 20, 30, 40) is in the exhausting mode, the inlet end (211) of the at least one air sterilization component (200) is connected to the main body (100).

3. The air sterilization mask (10, 20, 30, 40) according to claim 1, wherein the air sterilization mask (10, 20, 30, 40) comprises two air sterilization components (200), the air sterilization mask (10, 20, 30, 40) further has an exhaustion-inhalation mode, the outlet end (212) of the one of the two air sterilization components (200) is connected to the main body (100), and the inlet end (211) of another one of the two air sterilization components (200) is connected to the main body (100).

4. The air sterilization mask (10, 20, 30, 40) according to claim 1, wherein the casing (210) comprising:
a top cover (214), the top opening (2141) is located on the top cover (214);
a top body (215), having a top space (2151);
a bottom body (216), having a bottom space (2161); and
a bottom cover (217), the bottom opening (2171) is located on the bottom cover (217);
wherein, the top cover (214), the top body (215), the bottom body (216) and the bottom cover (217) are sequentially connected to one another, the top body (215) is removably connected to the bottom body (216), the main body (100) is selectively and removably connected to one of the top cover (214) and the bottom cover (217), the top opening (2141), the top space (2151), the bottom space (2161) and the bottom opening (2171) are connected to one another so as to together form the channel (213), the airflow generating unit (220) and the light sterilizer (230) are located in the top space (2151), and the airflow generating unit (220) is located closer to the top cover (214) than the light sterilizer (230).

5. The air sterilization mask (10, 20, 30, 40) according to claim 4, wherein the top body (215) further has an insertion hole (2152), the light sterilizer (230) has a charging plug (231), the insertion hole (2152) is connected to the top space (2151), and the charging plug (231) is disposed through the insertion hole (2152).

6. The air sterilization mask (10, 20, 30, 40) according to claim 4, wherein the bottom cover (217) comprises a cover body (2172) and a blocking sheet (2173), the bottom opening (2171) is located on the cover body (2172), the blocking sheet (2173) is disposed on a part of the cover body (2172) and spaced apart from another part of the cover body (2172), and the blocking sheet (2173) covers the bottom opening (2171).

7. The air sterilization mask (15) according to claim 4, wherein the bottom cover (217) comprises a cover body (2172) and a filter (2174), the bottom opening (2171) is located on the cover body (2172), and the filter (2174) is disposed on the cover body (2172) and occupies the bottom opening (2171).

8. The air sterilization mask (20) according to claim 1, wherein the main body (100) comprises a mouth-nose covering part (120) and an eye-forehead covering part (130) that are connected to each other, the accommodation space (110) comprises a mouth-nose accommodation space (111) and an eye-forehead accommodation space (112), the mouth-nose covering part (120) forms the mouth-nose accommodation space (111), the eye-forehead covering part (130) forms the eye-forehead accommodation space (112), the air sterilization component (200) is disposed on the mouth-nose covering part (120), and the channel (213) is in fluid communication with the mouth-nose accommodation space (111).

9. The air sterilization mask (30, 40) according to claim 1, wherein the main body (100) comprises a mouth-nose covering part (120), an eye-forehead covering part (130), two side parts (140, 150) and a top head-rear head covering part (113), the two side parts (140, 150) are respectively connected to two opposite sides of the mouth-nose covering part (120), two opposite sides of the eye-forehead covering part (130) and two opposite sides of the top head-rear head covering part (113), the mouth-nose covering part (120), the eye-forehead covering part (130), the two side parts (140, 150) and the top head-rear head covering part (113) together form the accommodation space (110), the accommodation space (110) comprises a mouth-nose accommodation space (111), an eye-forehead accommodation space (112) and a top head-rear head accommodation space (113) that are connected to one another, the mouth-nose covering part (120) forms the mouth-nose accommodation space (111), the eye-forehead covering part (130) forms the eye-forehead accommodation space (112), the two side parts (140, 150) and the top head-rear head covering part (113) together form the top head-rear head accommodation space (113), the two side parts (140, 150) each have an installing recess (141, 151) and a vent (142, 152), the two installing recesses (141, 151) are in fluid communication with the mouth-nose accommodation space (111), the two installing recesses (141, 151) are opposite to each other, the air sterilization mask (30, 40) comprises two air sterilization components (200), the two air sterilization component (200) are respectively located in the two installing recesses (141, 151), and the two channels (213) of the two air sterilization components (200) are respectively in fluid communication with the two vents (142, 152).

10. The air sterilization mask (40) according to claim 9, further comprising an enclosing component (400), wherein the main body (100) further has a wearing opening (190) that is together formed by the two side parts (140, 150), the mouth-nose covering part (120) and the top head-rear head covering part (113), the wearing opening (190) is in fluid communication with the top head-rear head accommodation space (113), the enclosing component (400) is disposed around the wearing opening (190).

11. The air sterilization mask (30, 40) according to claim 9, wherein the air sterilization mask (30, 40) has an inhaling mode, an exhausting mode and an exhaustion-inhalation mode, when the air sterilization mask (30, 40) is in the inhaling mode, the inlet ends (211) of the two air sterilization components (200) are respectively located closer to the two vents (142, 152) than the outlet ends (212) of the two air sterilization components (200), when the air sterilization mask (30, 40) is in the exhausting mode, the outlet ends (212) of the two air sterilization components (200) are respectively located closer to the two vents (142, 152) than the inlet ends (211) of the two air sterilization components (200), when the air sterilization mask (30, 40) is in the exhaustion-inhalation mode, the outlet end (212) of one of the two air sterilization components (200) is located closer to one of the two vents (142, 152) than the inlet end (211) of the one of the two air sterilization components (200), and the inlet end (211) of another one of the two air sterilization components (200) is located closer to another one of the two vents (142, 152) than the outlet end (212) of the another one of the two air sterilization components (200).

12. The air sterilization mask (30, 40) according to claim 11, wherein the casing (210) of each of the air sterilization components (200) comprising:
a top cover (214), the top opening (2141) located on the top cover (214);
a top body (215), having a top space (2151) and an insertion hole (2152) that are connected to each other;
a bottom body (216), having a bottom space (2161); and
a bottom cover (217), the bottom opening (2171) located on the bottom cover (217);
wherein, in each of the air sterilization components (200), the top cover (214), the top body (215), the bottom body (216) and the bottom cover (217) are sequentially connected to one another, the top body (215) is removably connected to the bottom body (216), the main body (100) is selectively and removably connected to one of the top cover (214) and the bottom cover (217), the top opening (2141), the top space (2151), the bottom space (2161) and the bottom opening (2171) are connected to one another so as to together form the channel (213), the airflow generating unit (220) and the light sterilizer (230) are located in the top space (2151), and the airflow generating unit (220) is located closer to the top cover (214) than the light sterilizer (230), the light sterilizer (230) has a charging plug (231) disposed through the insertion hole (2152); and
wherein the two side parts (140, 150) each have a first insertion hole (170) and a second insertion hole (180), in each of the two side parts (140, 150), the first insertion hole (170) and the second insertion hole (180) are connected to the installing recess (141, 151), and the first insertion hole (170) is located on a side of the second insertion hole (180) that is located away from the vent (142, 152), and;
wherein when the air sterilization mask (30, 40) is in the inhaling mode, the second insertion hole (180)s are respectively aligned with the two insertion hole (2152)s of the two top bodies (215), when the air sterilization mask (30, 40) is in the exhausting mode, the first insertion holes (170) are respectively aligned with the two insertion holes (2152) of the top bodies (215), when the air sterilization mask (30, 40) is in the exhaustion-inhalation mode, one of the second insertion holes (180) is aligned with one of the insertion holes (2152) of one of the two top bodies (215), and one of the first insertion holes (170) is aligned with one of the two insertion holes (2152) of one of the two top bodies (215).
